# EUROPÄISCHE PATENTSCHRIFT

(11) **EP 1 792 592 B1**
(45) Veröffentlichungstag und Bekanntmachung des Hinweises auf die Patenterteilung: **14.09.2011**
(21) Anmeldenummer: 05026259.1
(22) Anmeldetag: 01.12.2005
(51) Int. Cl.: A61F 9/01

(54) **Anordnung zur Durchführung chirurgischer Laserbehandlungen des Auges**
Arrangement for carrying out surgical laser treatments of the eye
Dispositif permettant des traitements chirurgicaux occulaires à l'aide d'un laser

(43) Veröffentlichungstag der Anmeldung: 06.06.2007
(73) Patentinhaber: WaveLight GmbH, 91058 Erlangen (DE)
(72) Erfinder: Donitzky, Christof, 90542 Eckental (DE); Vogler, Klaus, Dr., 90542 Eckental (DE)
(74) Vertreter: von Hellfeld, Axel

(56) Entgegenhaltungen:
- EP-A- 1 537 841
- US-A- 5 144 630
- US-A1- 2001 037 105
- US-A1- 2002 095 142
- US-A1- 2005 085 800

## Beschreibung

Die Erfindung betrifft eine Anordnung zur Durchführung chirurgischer Laserbehandlungen des Auges.

In der ophthalmologischen Chirurgie kommt gepulste Laserstrahlung verschiedener Wellenlängen zur Anwendung. Verschiedene Operationsvorgänge oder verschiedene Arten der Behandlung können unterschiedliche Laserwellenlängen erfordern, oder anders ausgedrückt kann für einen Operationsvorgang oder eine Behandlungsart eine Wellenlänge besser geeignet sein, während für einen anderen Operationsvorgang oder eine andere Behandlungsart eine andere Wellenlänge besser geeignet ist. Für die Anbringung von Schnitten in der Hornhaut beispielsweise ist es üblich, Laserstrahlung im kurzwelligen Infrarotbereich zu verwenden, etwa im Bereich zwischen 1000 nm und 1100 nm. Solche Schnitte können erforderlich sein beispielsweise bei einer LASIK-Operation zur Erzeugung eines zur Seite klappbaren Hornhautscheibchens (Flap) oder im Rahmen einer Korneatransplantation. Dagegen wird Laserstrahlung im ultravioletten Bereich häufig für den gezielten Abtrag von Material von der Hornhaut oder anderen Gewebestrukturen des Auges verwendet, etwa bei der Neuformung der Kornea zur Behebung einer Fehlsichtigkeit oder bei der Entfernung von Schadstellen im Hornhautgewebe. Typisch für den kornealen Materialabtrag ist beispielsweise eine Wellenlänge von 193 nm, die mittels eines Excimerlasers, etwa eines ArF-Lasers, erzeugt werden kann.

In manchen Fällen umfasst eine Operation verschiedene Vorgänge, die jeweils den Einsatz von Laserstrahlung einer unterschiedlichen Wellenlängen verlangen. LASIK-Operationen beispielsweise umfassen in der Regel die Anbringung eines Hornhautschnitts, wofür üblicherweise kurzwellige Infrarotstrahlung verwendet wird, und die anschließende Neuformung der Hornhaut durch Ablation, wofür gängigerweise UV-Strahlung eingesetzt wird. Herkömmlicherweise sind die Operationspraxen, in denen solche LASIK-Operationen durchgeführt werden, mit zwei vollständig getrennten Lasersystemen ausgestattet, nämlich einem mit einem IR-Laser und einem anderen mit einem UV-Laser. Jedes der Lasersysteme ist dabei mit allen Komponenten und Funktionen ausgestattet, um autark zu arbeiten, also unabhängig vom jeweils anderen Lasersystem. Das heißt, jedes Lasersystem enthält seine eigene Betriebsenergieversorgung, seine eigene Steuerung, seine eigenen Optikmittel für die Strahlformung und -führung sowie gegebenenfalls seine eigene Kühlung.

Es ist leicht vorstellbar, dass solche Lasersysteme Apparaturen beträchtlicher Größe sind. Aufgrund ihrer Ausmaße und ihres Gewichts sind sie in den Operationspraxen stationär aufgestellt, und zwar räumlich getrennt voneinander, weswegen der Patient zwischen Operationsschritten, bei denen die verschiedenen Lasersysteme zum Einsatz gelangen sollen, umgelagert werden muss. Dies ist ungünstig, insbesondere weil Augenbehandlungen am besten in einem kontinuierlichen, ununterbrochenen Vorgang durchgeführt werden sollten, und zwar in möglichst kurzer Zeit. Die Umlagerung des Patienten mitten in dem chirurgischen Eingriff ist für den Patienten äußerst unangenehm und stellt ein zusätzliches Risiko dar, denn zu diesem Zeitpunkt kann er mit dem zu behandelnden Auge nicht mehr sehen. Auch für den Operateur ist die Umlagerung des Patienten eine unerwünschte Unterbrechung im Operationsfluss, die womöglich seine Konzentration beeinträchtigen und damit das Operationsergebnis gefährden kann.

Die US 5,144,630 offenbart ein System mit einem Festkörperlaser und zusätzlichen Frequenzwandlern. Diese Frequenzwandler können aus einem oder mehreren nichtlinearen Kristallen bestehen. Spezielle Wellenlängen können durch Strahlteiler ausgewählt werden und mittels eines Zuführsystems zum Target geliefert werden. Bei einer Ausführungsform werden Wellenlängen im nahen Infrarotbereich, grünen Bereich und Ultraviolettbereich erzeugt. Bei einer anderen Ausführungsform werden Wellenlängen im Infrarotbereich erzeugt.

Die EP 1 537 841 A2 offenbart ein medizinisches System mit einem durchstimmbaren Laser.

Die US 2005/0085800 A1 offenbart einen Pumplaser, der eine erste Laserstrahlquelle und eine zweite Laserstrahlquelle anregt, die beide aus einem Ti:Saphir-Laser bestehen. Eine Frequenzwandlung des Ti:Saphir-Laserstrahls ermöglicht, dass ein Bereich unter 210 nm verwendet wird, indem der umgewandelte Ti:Saphir-Laserstahl mit 840 nm verdoppelt wird oder indem eine sequenzielle Summenfrequenz von 750nm mit einem Strahl von weniger als 200 nm gemischt wird.

Die US 2001/0037105 A1 beschreibt, wie ein Laserstrahl mit einer Wellenlänge von 1064 nm durch einen Verdoppelungskristall in einen grünen Strahl gewandelt wird und anschließend durch Mischen auf eine Wellenlänge 213 nm gewandelt wird.

Die US 2002/0095142 A1 offenbart Kristalle aus Lithiumborat (LBO) und Cäsium-Lithiumborat (CLBO), um einen Laserstrahl mit einer Wellenlänge von 1064 nm auf eine Wellenlänge von 213 nm zu wandeln.

Aufgabe der Erfindung ist es, eine Anordnung zur Durchführung chirurgischer Laserbehandlungen des Auges bereitzustellen, die einen vereinfachten Operationsablauf und infolgedessen eine erhöhte Gewähr für gute Behandlungsergebnisse bieten kann.

Die Aufgabe der Erfindung wird durch eine Vorrichtung gemäß Anspruch 1 gelöst.

Eine solche Anordnung umfasst eine gepulste Laserstrahlung einer ersten Wellenlänge bereitstellende Laserstrahlungsquelle sowie Frequenzwandlermittel, welche dazu eingerichtet sind, unter Verwendung der Laserstrahlung der ersten Wellenlänge gepulste Laserstrahlung mindestens einer weiteren Wellenlänge zu erzeugen, wobei die Anordnung dazu eingerichtet ist, Behandlungsstrahlung wahlweise mindestens zweier verschiedener Wellenlängen abzugeben.

Weil eine Behandlungsanordnung Laserstrahlung wahlweise verschiedener Wellenlängen bereitstellen kann, ist eine Umlagerung des Patienten zwischen Operationsschritten, die diese verschiedenen Wellenlängen erfordern, nicht mehr erforderlich. Der Operationsablauf wird hierdurch vereinfacht und verkürzt, was der Behandlungsqualität zu Gute kommt. Zudem benötigt eine erfindungsgemäße Behandlungsanordnung weniger Aufstellfläche als getrennte Lasersysteme. Die Erfindung ermöglicht auch Synergien, da im Unterschied zu getrennten Lasersystemen nur eine Betriebsenergieversorgungseinheit, eine Steuereinheit und ggf. auch nur ein Kühlaggregat für die einzige Laserstrahlungsquelle benötigt wird.

Gemäß einer Ausführungsform kann die Anordnung dazu eingerichtet sein, Behandlungsstrahlung wahlweise der ersten Wellenlänge oder mindestens einer von den Frequenzwandlermitteln erzeugten weiteren Wellenlänge abzugeben. Gemäß einer anderen Ausführungsform können dagegen die Frequenzwandlermittel dazu eingerichtet sein, unter Verwendung der Laserstrahlung der ersten Wellenlänge gepulste Laserstrahlung mindestens zweier weiterer, voneinander verschiedener Wellenlängen zu erzeugen, wobei die Anordnung Behandlungsstrahlung wahlweise zweier dieser weiteren Wellenlängen abgeben kann.

Vorzugsweise liegt die erste Wellenlänge zwischen etwa 800 nm und etwa 1200 nm, beispielsweise zwischen etwa 1000 nm und etwa 1100 nm oder sogar zwischen etwa 1030 nm und etwa 1070 nm. Die von den Frequenzwandlermitteln erzeugte mindestens eine weitere Wellenlänge liegt dagegen vorzugsweise zwischen etwa 190 nm und etwa 380 nm. Insbesondere können die Frequenzwandlermittel dazu eingerichtet sein, die erste Wellenlänge auf eine Wellenlänge zwischen etwa 190 nm und etwa 215 nm oder/und eine Wellenlänge zwischen etwa 340 nm und etwa 360 nm zu konvertieren. Bevorzugte weitere Wellenlängen sind etwa 193 nm und etwa 347 nm.

Die Pulsdauer der abgegebenen Behandlungsstrahlung liegt zumindest bei einer der von der Anordnung erzeugbaren Wellenlängen, insbesondere bei allen Wellenlängen, bevorzugt im Femto- oder Pikosekundenbereich. Gibt die Anordnung gepulste Behandlungsstrahlung im infraroten, insbesondere im niederen infraroten Wellenlängenbereich (z.B. zwischen 800 nm und 1200 nm) ab, so wird für die Pulsdauer der Behandlungsstrahlung ein Wert zwischen etwa 50 fs und etwa 800 fs, vorzugsweise zwischen etwa 100 fs und etwa 500 fs, höchstvorzugsweise zwischen etwa 100 fs und etwa 300 fs angestrebt. Bei abgegebener Behandlungsstrahlung im ultravioletten Wellenlängenbereich (z.B. zwischen 190 nm und 380 nm) wird dagegen bevorzugt eine Pulsdauer von höchstens etwa 500 ps, vorzugsweise höchstens etwa 10 ps und höchstvorzugsweise zwischen etwa 100 fs und etwa 500 fs gewählt. Es ist vorstellbar, dass unterschiedliche Pulsdauern für Behandlungsstrahlung unterschiedlicher Wellenlängen gewünscht wird. In diesem Fall kann die Anordnung Komponenten zur Pulsstreckung oder/und Pulskomprimierung umfassen, die bei Bedarf in Einsatz bringbar sein können. Solche Komponenten sind in der Fachwelt hinreichend bekannt und müssen hier nicht näher erläutert werden.

Die erfindungsgemäße Anordnung kann Mittel zur Fokussierung der Behandlungsstrahlung auf oder in einer Kornea umfassen. Der Fokusdurchmesser beträgt dabei im Fall von abgegebener Behandlungsstrahlung einer Wellenlänge zwischen etwa 800 nm und etwa 1200 nm oder zwischen etwa 340 nm und etwa 360 nm vorzugsweise höchstens etwa 10 µm, besser sogar höchstens etwa 5 µm. Im Fall von abgegebener Behandlungsstrahlung einer Wellenlänge zwischen etwa 190 nm und etwa 215 nm beträgt der Fokusdurchmesser dagegen vorzugsweise höchstens etwa 1 mm, besser sogar höchstens etwa 0,2 mm. Wellenlängen im infraroten Bereich sowie zwischen etwa 340 nm und etwa 360 nm eignen sich sehr gut für die Anbringung von Schnitten in der Hornhaut, etwa für die Flap-Erzeugung, während Wellenlängen zwischen etwa 190 nm und etwa 215 nm für die Neuformung der Hornhaut durch Photoablation besonders geeignet sind. Die erfindungsgemäße Anordnung ist deswegen bevorzugt dazu eingerichtet, im Rahmen einer Schnittbehandlung der Kornea Behandlungsstrahlung mit einer Wellenlänge zwischen etwa 800 nm und etwa 1200 nm oder zwischen etwa 340 nm und etwa 360 nm abzugeben, im Rahmen einer Behandlung für die Neuformung der Kornea jedoch Behandlungsstrahlung mit einer Wellenlänge zwischen etwa 190 nm und etwa 215 nm abzugeben.

Die Pulsrepetitionsrate der abgegebenen Behandlungsstrahlung kann beispielsweise zwischen etwa 5 kHz und etwa 500 kHz liegen, vorzugsweise zwischen etwa 10 kHz und etwa 300 kHz. Es ist freilich nicht ausgeschlossen, Pulsrepetitionsraten über 500 kHz bis hin in den MHz-Bereich zu verwenden, sogar bis in den zweistelligen MHz-Bereich, z.B. etwa 20 MHz.

Liegt die Wellenlänge der abgegebenen Behandlungsstrahlung im infraroten, insbesondere niederen infraroten Bereich, so liegt die Pulsenergie der Behandlungsstrahlung beispielsweise zwischen etwa 100 nJ und etwa 100 µJ, vorzugsweise zwischen etwa 1 µJ und etwa 10 µJ. Bei Wellenlängen der abgegebenen Behandlungsstrahlung im ultravioletten Bereich kann dagegen die Pulsenergie der Behandlungsstrahlung beispielsweise zwischen etwa 0,1 nJ und etwa 100 µJ liegen und insbesondere nicht mehr als einige wenige µJ betragen.

Die Fluence, d.h. die Energiedichte eines Pulses, der Behandlungsstrahlung kann insbesondere bei Wellenlängen im ultravioletten Bereich beispielsweise zwischen etwa 20 mJ/cm² und etwa 5 J/cm² betragen, wobei für Wellenlängen im Bereich zwischen 190 und 215 nm vorzugsweise eine Fluence zwischen 50 mJ/cm² und 500 m3/cm² eingestellt werden sollte, während für Wellenlängen im Bereich zwischen 340 und 360 nm vorzugsweise eine Fluence zwischen 0,5 J/cm² und 2 J/cm² eingestellt werden sollte.

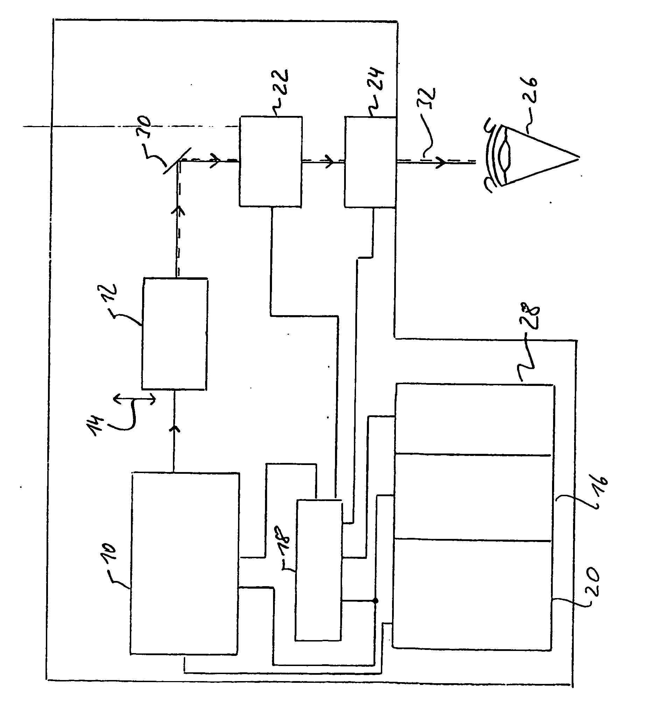

Die Erfindung wird nachstehend anhand der beigefügten einzigen Zeichnung weiter erläutert. Diese stellt schematisch ein Blockschaltbild eines Ausführungsbeispiels einer erfindungsgemäßen Behandlungsanordnung dar.

Die Behandlungsanordnung umfasst eine Laserstrahlungsquelle 10, welche gepulste Laserstrahlung mit Pulsdauern im Femtosekundenbereich emittiert. Die Wellenlänge der von der Laserstrahlungsquelle 10 emittierten Strahlung liegt im kurzwelligen infraroten Bereich, vorzugsweise zwischen 1000 nm und 1080 nm, beispielsweise bei 1040 nm oder 1064 nm. Es kann sich bei der Laserstrahlungsquelle 10 um einen Faserlaser handeln, wobei selbstverständlich andere Lasertypen denkbar sind. Im optischen Weg der von der Laserstrahlungsquelle 10 emittierten Strahlung ist schematisch eine Frequenzwandleranordnung 12 gezeigt, welche dazu eingerichtet ist, eine Frequenzkonversion der Infrarot-Strahlung in den UV-Bereich durchzuführen, nämlich auf mindestens eine Wellenlänge im Bereich zwischen 190 nm und 380 nm. Vorzugsweise kann die Frequenzwandleranordnung 12 zumindest eine Wellenlänge zwischen 190 nm und 215 nm erzeugen, beispielsweise 193 nm, gewünschtenfalls auch wahlweise eine weitere Wellenlänge zwischen 340 nm und 360 nm, beispielsweise 347 nm.

Die Behandlungsanordnung kann gepulste Laserstrahlung wahlweise mindestens zweier verschiedener Wellenlängen abgeben. Eine der abgebbaren Wellenlängen ist vorzugsweise eine Wellenlänge zwischen 190 nm und 215 nm. Eine andere abgebbaren Wellenlänge kann unmittelbar die IR-Wellenlänge der Laserstrahlungsquelle 10 sein oder/und eine Wellenlänge zwischen 340 nm und 360 nm. Soll Behandlungsstrahlung mit der IR-Wellenlänge der Laserstrahlungsquelle 10 abgegeben werden, ist keine Frequenzkonversion durch die Frequenzwandleranordnung 12 erforderlich. Die Frequenzwandleranordnung 12 kann hierfür so in die Behandlungsanordnung eingebaut sein, dass sie nach Wunsch als eine Einheit oder ein Modul in den optischen Weg der von der Laserstrahlungsquelle 10 emittierten Laserstrahlung hineinbewegbar und wieder herausbewegbar ist, wie in der Figur durch einen Doppelpfeil 14 angedeutet. Die Frequenzwandleranordnung 12 kann hierbei beispielsweise entlang einer linearen Bahn oder entlang einer Schwenkbahn verstellbar sein. Eine präzise Bewegungsführung der Frequenzwandleranordnung 12 kann durch nicht näher dargestellte Führungsmittel gewährleistet sein. Beispielsweise kann die Frequenzwandleranordnung 12 per Hand oder mittels einer elektromotorischen Antriebseinheit bewegbar sein. Anschlag- oder Rastmittel oder/und eine geeignete Sensorik können dabei eine exakte Positionierung des Frequenzwandleranordnung 12 entlang ihrer Verstellbarkeitsbahn erleichtern.

Soll die Behandlungsanordnung Behandlungsstrahlung wahlweise zweier verschiedener UV-Wellenlängen abgeben können, so kann hierfür die Frequenzwandleranordnung 12 beispielsweise zwei Wandlereinheiten umfassen, deren jede die von der Laserstrahlungsquelle 10 erzeugte IR-Wellenlänge auf je eine der UV-Wellenlängen wandeln kann und die jeweils nach Wunsch entweder die eine oder die andere in den optischen Weg der von der Laserstrahlungsquelle 10 bereitgestellten Laserstrahlung bewegbar sind. In einem solchen Fall ist es vorstellbar, die beiden Wandlereinheiten mechanisch miteinander zu koppeln, wobei je nach Stellung der Frequenzwandleranordnung 12 entlang ihrer Verstellbarkeitsbahn entweder die eine oder die andere der Wandlereinheiten oder möglicherweise keine der Wandlereinheiten wirksam ist. Es ist selbstverständlich auch möglich, dass die beiden Wandlereinheiten unabhängig voneinander in und aus den optischen Weg der Laserstrahlung bewegbar sein können.

Zur Erzeugung der einen oder mehreren UV-Wellenlängen aus der von der Laserstrahlungsquelle 10 bereitgestellten IR-Wellenlänge kann die Frequenzwandleranordnung 12 in der Fachwelt an sich bekannte optische Komponenten wie beispielsweise optisch parametrische Generatoren/Oszillatoren, Summenfrequenzgeneratoren und Generatoren für die zweite, dritte, vierte oder fünfte Harmonische umfassen oder/und sich spezieller Schemata für die Summenfrequenzbildung bedienen. Konzepte zur Frequenzkonversion sind dem Fachmann wohl vertraut, weswegen hierauf nicht näher eingegangen werden muss.

Die Behandlungsanordnung umfasst ferner eine Betriebsenergieversorgungseinheit 16, welche die Laserstrahlungsquelle 10, eine Steuereinheit 18 sowie gewünschtenfalls weitere Komponenten der Behandlungsanordnung mit Betriebsenergie versorgt. Eine optional vorgesehene Kühleinheit 20 kann eine Kühlung der Laserstrahlungsquelle 10 bewirken. Die Steuereinheit 18 steuert den Betrieb der Laserstrahlungsquelle 10 sowie gewünschtenfalls eine im Strahlengang nach der Frequenzwandleranordnung 12 angeordnete Ablenkeinheit 22 und eine nachgeschaltete Fokussiereinheit 24. Die Ablenkeinheit 22 (auch als Scanner bezeichnet) gestattet eine solche Lenkung der Laserstrahlung, dass der gewünschte Zielbereich beispielsweise in aufeinanderfolgenden Linien überstrichen wird. Die Fokussiereinheit 24 fokussiert die Laserstrahlung auf einen kornealen oder intraokularen Zielpunkt eines zu behandelnden Auges, welches schematisch bei 26 angedeutet ist. Eine Recheneinheit 28 berechnet für die Durchführung der Laserbehandlung erforderliche Daten wie beispielsweise ein Ablationsprofil oder ein Schnittprofil und liefert diese Daten an die Steuereinheit 18, welche auf Grundlage der Daten den Betrieb der Komponenten 10 oder/und 22 oder/und 24 entsprechend steuert.

Es versteht sich, dass die Behandlungsanordnung zusätzlich noch weitere optische Komponenten für die Führung der Laserstrahlung enthalten kann, wie beispielhaft durch einen Umlenkspiegel 30 angedeutet.

Die Laserstrahlungsquelle 10, die Frequenzwandleranordnung 12, die Ablenkeinheit 22 sowie die Fokussiereinheit 24 sind vorzugsweise in einem gemeinsamen Gehäuse untergebracht, so dass sich insgesamt eine kompakte, vielfältig einsetzbare Behandlungsanordnung ergibt. Gewünschtenfalls können auch die übrigen Komponenten der Behandlungsanordnung, insbesondere die Steuereinheit 18, die Recheneinheit 28, die Betriebsenergieversorgungseinheit 16 sowie die Kühleinheit 20, wenigstens teilweise in demselben Gehäuse zusammen mit der Laserstrahlungsquelle 10 untergebracht sein. Alternativ können sie in einem oder mehreren gesonderten Gehäusen untergebracht sein, die jedoch fest mit dem die Laserstrahlungsquelle 10 enthaltenden Gehäuse verbunden sein können.

Als Alternative zu einem linear oder schwenkbeweglichen Einbau der Frequenzwandleranordnung 12 ist es vorstellbar, diese unbeweglich einzubauen und zwischen der Laserstrahlungsquelle 10 und der Frequenzwandleranordnung 12 eine verstellbare Umlenkspiegelanordnung vorzusehen, abhängig von deren Position oder/und Orientierung die von der Laserstrahlungsquelle 10 emittierte Laserstrahlung entweder zu der Frequenzwandleranordnung 12 weitergeleitet wird oder an dieser vorbeigeleitet wird. Eine solche verstellbare Umlenkspiegelanordnung kann beispielsweise mindestens einen linear oder entlang einer anderen Bahn in den Strahlengang der von der Laserstrahlungsquelle 10 emittierten Laserstrahlung hineinbewegbaren und wieder herausbewegbaren Umlenkspiegel oder/und mindestens einen schwenkbeweglichen Umlenkspiegel umfassen.

Die von der Behandlungsanordnung letztendlich abgegebenen Behandlungspulse - in der Zeichnung mit 32 bezeichnet - können beispielsweise Parameter wie folgt besitzen. Bei einer Wellenlänge im Bereich zwischen 1000 nm und 1100 nm kann die Pulslänge zwischen 50 fs und 800 fs betragen, vorzugsweise zwischen 100 fs und 500 fs und insbesondere zwischen 100 fs und 300 fs. Die Pulsenergie kann zwischen 100 nJ und 100 µJ betragen, vorzugsweise zwischen 1 µJ und 10 µJ. Hierbei wird zur Vermeidung zu großer Kavitationsblasen ein möglichst kleiner Fokusquerschnitt angestrebt, beispielsweise mit einem Durchmesser von höchstens 10 µm, vorzugsweise höchstens 5 µm. Die Pulsrepetitionsrate kann zwischen 5 kHz und 500 kHz, vorzugsweise zwischen 20 kHz und 300 kHz liegen. Bei solchen vergleichsweise hohen Pulsrepetitionsraten ist eine sehr rasche Anbringung eines Hornhautschnitts möglich.

Wird die Behandlungsanordnung im Frequenzwandlungsmodus betrieben und liegt die Wellenlänge der Behandlungspulse 32 dementsprechend im UV-Bereich, so beträgt die Pulsenergie vorzugsweise zwischen etwa 0,5 nJ und etwa 5 µJ. Die Pulsrepetitionsrate kann beispielsweise im dreistelligen kHz-Bereich bis hin in den MHz-Bereich liegen. Die Pulsenergie und die Pulsrepetitionsrate werden zweckmäßigerweise so aufeinander abgestimmt sein, dass die integrierte Energie ausreicht, um die Ablationsschwelle des zu behandelnden Gewebes zu überschreiten.

Bei Verwendung von Behandlungspulsen 32 mit UV-Wellenlängen ist es grundsätzlich denkbar, längere Pulsdauern einzusetzen, als sie von der Laserstrahlungsquelle 10 erzeugt werden. Die Behandlungsanordnung kann hierzu mit in der Fachwelt an sich bekannten Komponenten zur Pulsdilatation ausgestattet sein, wobei diese Komponenten beispielsweise in der Frequenzwandleranordnung 12 enthalten sein können oder in einem gesonderten, jedoch mit der Frequenzwandleranordnung 12 gekoppelten Pulsstreckungsmodul untergebracht sein können, so dass sie nur bei Frequenzkonvertierung der von der Laserstrahlungsquelle 10 erzeugten IR-Wellenlänge zum Einsatz kommen. Es ist selbstverständlich auch möglich, auf solche Pulsdilatationsmittel zu verzichten, so dass sowohl im IR-Bereich als auch im UV-Bereich gleiche Pulsdauern im Femtosekundenbereich zum Einsatz kommen.

## Patentansprüche

1. Hornhautschnitt-Hornhautablation-Anordnung zur Durchführung chirurgischer Laserbehandlungen des Auges, mit
- einer gepulste Laserstrahlung einer ersten Wellenlänge bereitstellenden Laserstrahlungsquelle (10);
- Frequenzwandlermitteln (12), welche dazu eingerichtet sind, unter Verwendung der Laserstrahlung der ersten Wellenlänge gepulste Laserstrahlung mindestens einer weiteren Wellenlänge zu erzeugen;
- einer Ablenkeinheit (22), die die Laserstrahlung der ersten oder weiteren Wellenlänge ablenkt;
- einer Fokussiereinheit (24), die die Laserstrahlung der ersten oder weiteren Wellenlänge auf einen komealen Zielpunkt fokussiert;
- einer Recheneinheit (28); und
- einer Steuereinheit (18);
wobei die Anordnung dazu eingerichtet ist, Behandlungsstrahlung (32) wahlweise der ersten Wellenlänge oder mindestens einer von den Frequenzwandlern erzeugten weiteren Wellenlänge abzugeben, **gekennzeichnet dadurch, dass**
die Recheneinheit (28) an die Steuereinheit ein Schnittprofil, falls ein Schnitt in der Kornea durchgeführt wird, oder ein Ablationsprofil, falls eine Ablation der Komea durchgeführt wird, übergibt und die Steuereinheit (18) die Ablenkeinheit (22) und/oder Fokussiereinheit (24) entsprechend dem Schnittprofil bzw. dem Ablationsprofil steuert.

2. Hornhautschnitt-Hornhautablation-Anordnung nach Anspruch 1 oder 2,
**dadurch gekennzeichnet, dass** die Frequenzwandlermittel dazu eingerichtet sind, unter Verwendung der Laserstrahlung der ersten Wellenlänge gepulste Laserstrahlung mindestens zweier weiterer, voneinander verschiedener Wellenlängen zu erzeugen, und dass die Anordnung dazu eingerichtet ist, Behandlungsstrahlung (32) wahlweise zweier von den Frequenzwandlermitteln erzeugter weiterer Wellenlängen abzugeben.

3. Hornhautschnitt-Hornhautablation-Anordnung nach einem der vorhergehenden Ansprüche,
**dadurch gekennzeichnet, dass** die erste Wellenlänge zwischen etwa 800 nm und etwa 1200 nm liegt, vorzugsweise zwischen etwa 1000 nm und etwa 1100 nm, höchstvorzugsweise zwischen etwa 1030 nm und etwa 1070 nm, und dass die mindestens eine weitere Wellenlänge zwischen etwa 190 nm und etwa 380 nm liegt.

4. Hornhautschnitt-Hornhautablation-Anordnung nach Anspruch 3,
**dadurch gekennzeichnet, dass** eine von den Frequenzwandlermitteln erzeugte weitere Wellenlänge zwischen etwa 190 nm und etwa 215 nm liegt, vorzugsweise bei etwa 193 nm.

5. Hornhautschnitt-Hornhautablation-Anordnung nach Anspruch 3 oder 4,
**dadurch gekennzeichnet, dass** eine von den Frequenzwandlermitteln erzeugte weitere Wellenlänge zwischen etwa 340 nm und etwa 360 nm liegt, vorzugsweise bei etwa 347 nm.

6. Hornhautschnitt-Hornhautablation-Anordnung nach einem der vorhergehenden Ansprüche,
**dadurch gekennzeichnet, dass** die Pulsdauer der Behandlungsstrahlung (32) zumindest einer der Wellenlängen im Femto- oder Pikosekundenbereich liegt.

7. Hornhautschnitt-Hornhautablation-Anordnung nach einem der Ansprüche 3 bis 6
**dadurch gekennzeichnet, dass** die Fokussiereinheit (24) so eingerichtet ist, dass der Fokusdurchmesser im Fall von abgegebener Behandlungsstrahlung einer Wellenlänge zwischen etwa 800 nm und etwa 1200 nm oder zwischen etwa 340 nm und etwa 360 nm höchstens etwa 10 µm beträgt, vorzugsweise höchstens etwa 5 µm, und/oder dass der Fokusdurchmesser im Fall von abgegebener Behandlungsstrahlung einer Wellenlänge zwischen etwa 190 nm und etwa 215 nm höchstens etwa 1 mm beträgt, vorzugsweise höchstens etwa 0,2 mm.

8. Hornhautschnitt-Hornhautablation-Anordnung nach einem der vorangegangenen Ansprüche,
**gekennzeichnet durch** Impulskomprimierungsmittel und/oder Impulsstreckmittel.

## Claims

1. A cornea incision and cornea ablation arrangement for performing surgical laser treatment of the eye, having
- a laser radiation source (10) providing a pulsed laser radiation having a first wavelength;
- frequency converter means (12), which are arranged to generate by using the laser radiation of the first wavelength pulsed laser radiation having at least a further wavelength;
- a deflection unit (22) deflecting the laser radiation of the first or further wavelength;
- a focusing unit (24) focusing the laser radiation of the first or further wavelength to a corneal target spot;
- a computing unit (28); and
- a control unit (18);
wherein the arrangement is configured to selectively emit treatment radiation (32) having the first wavelength or having at least of one further wavelength generated by the frequency converters, **characterized in that**
the computing unit (28) transfers to the control unit a cutting profile, if a cut in the cornea is performed, or an ablation profile, if an ablation of the cornea is performed, and the control unit (18) controls the deflection unit (22) and/or the focusing unit (24) according to the cutting profile and the ablation profile, respectively.

2. The cornea incision and cornea ablation arrangement according to claim 1, **characterized in that** the frequency converter means are configured to generate pulsed laser radiation having at least two further wavelengths differing from each other using the laser radiation of the first wavelength, and that the arrangement is configured to selectively emit treatment radiation (32) having two further wavelengths generated by the frequency converter means.

3. The cornea incision and cornea ablation arrangement according to one of the preceding claims,
**characterized in that** the first wavelength ranges between approximately 800 nm to approximately 1200 nm, preferably between approximately 1000 nm to approximately 1100 nm, most preferably between approximately 1030 nm to approximately 1070 nm and that the at least one further wavelength ranges between approximately 190 nm to approximately 380 nm.

4. The cornea incision and cornea ablation arrangement according to claim 3, **characterized in that** the further wavelength generated by the frequency converter means ranges between approximately 190 nm to approximately 215 nm, preferably at approximately 193 nm.

5. The cornea incision and cornea ablation arrangement according to claim 3 or 4, **characterized in that** the further wavelength generated by the frequency converter means ranges between approximately 340 nm to approximately 360 nm, preferably at approximately 374 nm.

6. The cornea incision and cornea ablation arrangement according to one of the preceding claims,
**characterized in that** the pulse duration of the treatment radiation (32) of at least one of the wavelength ranges in the femto or pico second range.

7. The cornea incision and cornea ablation arrangement according to one of claims 3 to 6,
**characterized in that** the focusing unit (24) is configured such that the focus diameter is, in case of an emitted treatment radiation having a wavelength between approximately 800 nm to approximately 1200 nm or between approximately 340 nm to approximately 360 nm, not exceeding 10 µm, preferably not exceeding approximately 5 µm, and/or that the focus diameter is, in case of an emitted treatment radiation having a wavelength between approximately 190 nm to approximately 250 nm, not exceeding approximately 1 mm, preferably not exceeding approximately 0,2 mm.

8. The cornea incision and cornea ablation arrangement according to one of the preceding claims,
**characterized by** pulse compression means and/or pulse elongation means.

## Revendications

1. Dispositif pour l'ablation ou l'incision de la cornée permettant des traitements chirurgicaux oculaires à l'aide d'un laser, comprenant
- une source de rayonnement laser (10) pour la mise à disposition d'un rayonnement laser pulsé d'une première longueur d'onde,
- des moyens formant convertisseurs de fréquence (12), conçus pour produire, moyennant l'utilisation dudit rayonnement laser de la première longueur d'onde, un rayonnement laser pulsé au moins d'une autre longueur d'onde,
- une unité de déviation (22) qui dévie le rayonnement laser de la première ou de l'autre longueur d'onde,
- une unité de focalisation (24) qui focalise sur un point cornéen cible le rayonnement laser de la première ou de l'autre longueur d'onde,
- une unité de calcul (28) et
- une unité de commande (18),
le dispositif étant conçu pour émettre, au choix, un rayonnement de traitement (32) de la première longueur d'onde ou un rayonnement de traitement au moins d'une autre longueur d'onde produite par les convertisseurs de fréquence, **caractérisé en ce que**
l'unité de calcul (28) transmet à l'unité de commande un profil d'incision, en cas d'incision dans la cornée, ou un profil d'ablation, en cas d'ablation de la cornée, et l'unité de commande (18) commande l'unité de déviation (22) et/ou l'unité de focalisation (24) conformément au profil d'incision ou au profil d'ablation.

2. Dispositif pour l'ablation ou l'incision de la cornée selon la revendication 1,
**caractérisé en ce que** les moyens formant convertisseurs de fréquence sont conçus pour produire, moyennant l'utilisation du rayonnement laser de la première longueur d'onde, un rayonnement laser pulsé au moins de deux autres longueurs d'onde différentes l'une de l'autre, et **en ce que** le dispositif est conçu pour émettre un rayonnement de traitement (32), au choix, des deux autres longueurs d'onde produites par les moyens formant convertisseurs de fréquence.

3. Dispositif pour l'ablation ou l'incision de la cornée selon l'une des revendications précédentes,
**caractérisé en ce que** la première longueur d'onde est comprise entre environ 800 nm et environ 1200 nm, préférentiellement entre environ 1000 nm et environ 1100 nm, et encore plus préférentiellement entre environ 1030 nm et environ 1070 nm, et **en ce que** ladite autre longueur d'onde est comprise entre environ 190 nm et environ 380 nm.

4. Dispositif pour l'ablation ou l'incision de la cornée selon la revendication 3,
**caractérisé en ce qu'**une autre longueur d'onde produite par les moyens formant convertisseurs de fréquence est comprise entre environ 190 nm et environ 215 nm, étant préférentiellement d'environ 193 nm.

5. Dispositif pour l'ablation ou l'incision de la cornée selon la revendication 3 ou 4,
**caractérisé en ce qu'**une autre longueur d'onde produite par les moyens formant convertisseurs de fréquence est comprise entre environ 340 nm et environ 360 nm, étant préférentiellement d'environ 347 nm.

6. Dispositif pour l'ablation ou l'incision de la cornée selon l'une des revendications précédentes,
**caractérisé en ce que** la durée d'impulsion du rayonnement de traitement (32) au moins d'une des longueurs d'onde est située dans la plage des femtosecondes ou des picosecondes.

7. Dispositif pour l'ablation ou l'incision de la cornée selon l'une des revendications 3 à 6,
**caractérisé en ce que** l'unité de focalisation (24) est conçue de sorte que, dans le cas de l'émission d'un rayonnement de traitement d'une longueur d'onde comprise entre environ 800 nm et environ 1200 nm ou entre environ 340 nm et environ 360 nm, le diamètre du foyer s'élève à environ 10 µm maximum, préférentiellement à environ 5 µm maximum, et/ou **en ce que**, dans le cas de l'émission d'un rayonnement de traitement d'une longueur d'onde comprise entre environ 190 nm et environ 215 nm, le diamètre du foyer s'élève à environ 1 mm maximum, préférentiellement à environ 0,2 mm.

8. Dispositif pour l'ablation ou l'incision de la cornée selon l'une des revendications précédentes,
**caractérisé par** des moyens de compression d'impulsion et/ou des moyens d'extension d'impulsion.
